(19) **Europäisches Patentamt**
**European Patent Office**
**Office européen des brevets**

(11) **EP 2 975 983 B1**

(12) **FASCICULE DE BREVET EUROPEEN**

(45) Date de publication et mention
de la délivrance du brevet:
**12.04.2017 Bulletin 2017/15**

(51) Int Cl.:
*G01B 21/32* (2006.01)          *G01N 33/03* (2006.01)
*A47J 36/00* (2006.01)          *A47J 37/10* (2006.01)

(21) Numéro de dépôt: **14715350.6**

(22) Date de dépôt: **10.03.2014**

(86) Numéro de dépôt international:
**PCT/FR2014/050536**

(87) Numéro de publication internationale:
**WO 2014/147319 (25.09.2014 Gazette 2014/39)**

(54) **PROCÉDÉ DE DÉTERMINATION D'UN INDICE DE PERFORMANCE D'UN USTENSILE CULINAIRE POUR UNE TEMPÉRATURE DE CUISSON PRÉDETERMINÉE DANS LE BUT D'ÉVALUER UN GAIN NUTRITIONNEL**

VERFAHREN ZUR BESTIMMUNG EINES LEISTUNGSINDEX EINES KOCHGESCHIRRS FÜR EINE VORBESTIMMTE KOCHTEMPERATUR MIT DEM ZIEL DER BEURTEILUNG EINER NÄHRWERTERHÖHUNG

METHOD FOR DETERMINING A PERFORMANCE INDEX OF A COOKING UTENSIL FOR A PREDETERMINED COOKING TEMPERATURE WITH THE AIM OF ASSESSING NUTRITIONAL GAIN

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **20.03.2013 FR 1352473**

(43) Date de publication de la demande:
**27.01.2016 Bulletin 2016/04**

(73) Titulaire: **SEB S.A.**
**69130 Ecully (FR)**

(72) Inventeurs:
• **HOFLEITNER, Céline**
  **74000 Annecy (FR)**
• **CUILLERY, Pascal**
  **74210 Faverges (FR)**

(74) Mandataire: **Bourrières, Patrice**
**SEB Développement SAS**
**Campus SEB**
**112 Chemin du Moulin Carron**
**69134 Ecully Cedex (FR)**

(56) Documents cités:
**US-A1- 2010 224 636**

**Description**

La présente invention concerne le domaine des ustensiles culinaires, et tout particulièrement un procédé de détermination d'un indice de performance d'un ustensile culinaire pour une température de cuisson prédéterminée dans le but d'évaluer un gain nutritionnel, en particulier en fonction de la déformation du fond de l'ustensile culinaire. L'invention vise à montrer le rapport entre le gain nutritionnel des cuissons réalisées dans des ustensiles culinaires et la concavité du fond de ces ustensiles culinaires. L'invention concerne donc tous les ustensiles culinaires destinés à la cuisson d'aliments, notamment les poêles.

La fabrication des ustensiles culinaires pour la cuisson d'aliments, tels que par exemple des poêles, est telle que cet ustensile présente en sortie de fabrication, un fond concave. En effet, un ustensile culinaire avec un fond convexe engendrerait un défaut de stabilité de cet ustensile. Lors de la chauffe de l'ustensile culinaire, celui-ci est soumis à de fortes variations de température, qui entraînent des phénomènes de dilatation et de déformation. Le fond de l'ustensile culinaire peut alors changer de concavité, voire devenir convexe lors de la cuisson d'un aliment, ce changement de forme étant fonction d'une part, de sa concavité en sortie de fabrication et d'autre part, de la conception du fond de l'ustensile culinaire. La demanderesse a déjà développé des ustensiles culinaires pour la cuisson d'aliments qui présentent des caractéristiques techniques permettant de minimiser au maximum la déformation du fond de ces ustensiles lors d'une cuisson. De tels ustensiles culinaires sont par exemple décrits dans les demandes de brevet publiées sous les numéros FR 2 711 050, FR 2 780 626 et FR 2 919 484.

Le document US 2010/0224636 A1 décrit une méthode de test de la performance d'un ustensile culinaire.

L'homme du métier a également développé des procédés de mesure de la concavité des ustensiles culinaires aussi bien à froid qu'au cours de la cuisson, voire après vieillissement. Ainsi, par exemple, on peut mesurer la concavité à froid du fond de l'ustensile au moyen d'un comparateur. On peut mesurer la concavité du fond de l'ustensile au cours de la cuisson au moyen d'un comparateur et d'un thermocouple. On peut également mesurer le profil du fond de l'ustensile par laser.

L'homme du métier a également développé des ustensiles culinaires dont le fond présente une surface interne qui comprend un revêtement antiadhésif. Il n'en demeure pas moins que pour la cuisson de certains aliments, comme par exemple les pommes de terre préparées dans une poêle ou une sauteuse, le cuisinier utilise tout de même de la matière grasse qui est un réel outil technologique pour réussir la cuisson et permet d'améliorer la qualité organoleptique de l'aliment préparé ou cuisiné. D'un point de vue nutritionnel, il est en revanche préconisé de limiter la consommation de matière grasse, entre 35% à 40% de l'apport énergétique journalier.

L'objet de la présente invention est de déterminer un indice de performance d'un ustensile culinaire pour une température de cuisson prédéterminée et de caractériser le gain nutritionnel apporté par la maîtrise de la planéité du fond des ustensiles culinaire durant la cuisson des aliments, en corrélation avec la qualité organoleptique que recherche le cuisinier lors de la cuisson de ces aliments.

A cet effet, l'invention concerne un procédé de détermination d'un indice de performance d'un ustensile culinaire pour une température de cuisson prédéterminée dans le but d'évaluer un gain nutritionnel, ledit ustensile culinaire comprenant un fond présentant une surface de cuisson, caractérisé en ce qu'il comprend les étapes suivantes :

a) porter la surface de cuisson à ladite température de cuisson prédéterminée en utilisant des moyens de chauffe, l'échauffement de la surface entrainant une déformation du fond,
b) détermination d'une quantité de matière grasse minimale nécessaire pour recouvrir l'intégralité de la surface de cuisson du fond ainsi déformé à ladite température de cuisson prédéterminée en versant la quantité de matière grasse minimale nécessaire pour recouvrir l'intégralité de la surface de cuisson ou en calculant ladite quantité de matière grasse minimale nécessaire pour recouvrir l'intégralité de la surface de cuisson en recréant l'ustensile culinaire ainsi déformé à l'aide d'un ordinateur muni d'un logiciel de conception assistée par ordinateur, ladite quantité de matière grasse minimale permettant de définir l'indice de performance de l'ustensile culinaire pour une température de cuisson prédéterminée.

Ces étapes sont réalisées pour un panel d'ustensiles culinaires pour la cuisson des aliments, ces ustensiles culinaires étant d'un même type, par exemple des poêles d'un diamètre de 26 cm provenant de différents fabricants.

Ainsi, l'indice de performance de l'article culinaire pour une température de cuisson prédéterminée est déterminé par la quantité de matière grasse minimale. Le meilleur indice de performance pour une température de cuisson prédéterminée est obtenu avec l'article culinaire mettant en oeuvre la quantité de matière grasse minimale la plus faible. La quantité de matière grasse minimale est directement liée à la déformation du fond.

Dans un mode de mise en oeuvre préférentiel, pour réaliser l'étape b) on procède par étapes successives :

i. on verse une quantité de matière grasse correspondant à un volume de liquide de 1 mm d'épaisseur et de surface égale à un disque plan équivalent à la surface de cuisson,

ii. on laisse la matière grasse se stabiliser à la température et se répartir sur la surface de cuisson du fond,

iii. on mesure une surface de cuisson résiduelle non recouverte par la matière grasse et on verse une quantité de matière grasse correspondant à un volume de liquide de 1 mm d'épais et de surface égale à un disque plan équivalent à la surface de cuisson résiduelle

iv. on répète les étapes ii) et iii) jusqu'à recouvrir intégralement la surface de cuisson du fond de l'ustensile culinaire.

Ainsi la somme des quantités de matière grasse versées dans la poêle détermine la quantité d'huile minimale pour recouvrir intégralement le fond de l'article culinaire.

Dans une variante de mise en oeuvre, selon ce mode préférentiel, on créé l'ustensile culinaire en conception assistée par ordinateur, on représente graphiquement la quantité minimale de matière grasse à l'état liquide nécessaire pour recouvrir le fond de l'ustensile culinaire puis, on analyse les propriétés massiques de l'objet créé en conception assistée par ordinateur en vue de calculer ladite quantité de liquide nécessaire pour la cuisson.

Avantageusement, à l'étape a) la température de cuisson prédéterminée choisie est celle de la pomme de terre, de l'ordre de 180 à 200°C.

Le choix de la température de cuisson prédéterminée correspond au choix d'un aliment à cuire qui est de préférence orienté vers des aliments ou des recettes qui sont couramment cuisinés et qui nécessitent un apport en matière grasse. Idéalement on choisira un aliment cuisiné en friture plate, c'est-à-dire avec un mince film de matière grasse, à l'état liquide lorsqu'elle est chauffée, par opposition à la friture profonde où l'aliment est plongé dans la matière grasse liquide. Ainsi, de manière préférentielle et non limitative, on choisit comme aliment à cuire des pommes de terre en morceaux, en rondelles ou entières, selon leur grosseur, pour la réalisation de la recette des pommes de terre sautées.

Dans un mode de réalisation préférentiel et non limitatif, la matière grasse est de l'huile alimentaire. On pourrait toutefois envisager l'utilisation de beurre ou similaire, voire de gras de viande, ou autres.

Avantageusement, le procédé comprend les étapes suivantes :

a) cuisson de l'aliment dans l'ustensile culinaire et quantification de la matière grasse absorbée par l'aliment cuit ;
b) détermination du taux de matière grasse absorbée par rapport à la matière sèche de l'aliment cuit.

Dans un mode de réalisation préférentiel et non limitatif de l'étape c), on mesure la masse $m_{uc}$ de l'ustensile culinaire, la masse $m_{mg}$ de la matière grasse utilisée, la masse $m_{ac}$ de l'aliment cru, la masse $m_{ap}$ de l'aliment préparé, la masse $m_{uc+rmg}$ de l'ustensile culinaire et du résidu de matière grasse une fois l'aliment préparé, retiré de l'ustensile culinaire et on en déduit la masse $m_{mga}$ de la matière grasse absorbée par l'aliment préparé.

Avantageusement, le taux de matière grasse absorbée par rapport à la matière sèche de l'aliment cuit est déterminé par la formule $Tx_{mga/ms} = M_{mga}/m_{ac}/Tx_{ms}$ où $Tx_{ms}$ est le taux de matière sèche de l'aliment.

De manière préférentielle, le procédé comporte une étape de mesure de la déformation du fond de l'ustensile culinaire à la température de cuisson prédéterminée après l'étape a) et à l'étape d), on détermine le taux de matière grasse absorbée par rapport à la matière sèche de l'aliment cuit en fonction de la déformation de l'ustensile culinaire.

Diverses techniques de mesure de la déformation du fond, connues ou non de l'homme du métier, pourront être mises en oeuvre dans le cadre de l'invention. De manière préférentielle et non limitative, la mesure de la déformation du fond de l'ustensile culinaire à une température de cuisson prédéterminée est réalisée conformément à la norme XP D21-503.

Dans un mode préférentiel de mise en oeuvre du procédé selon l'invention, l'ustensile culinaire est une poêle. On peut envisager des variantes de mise en oeuvre avec d'autres ustensiles culinaires pour la cuisson d'aliments, comme par exemple une sauteuse, une casserole ou un fait-tout.

La description suivante concerne un mode préférentiel de mise en oeuvre du procédé selon l'invention, laquelle s'appuie sur des figures parmi lesquelles :

- Les figures 1 et 2 montrent deux ustensiles culinaires pour la cuisson d'aliments dont les fonds présentent respectivement une concavité faible et une concavité élevée ;
- La figure 3 illustre des moyens pour déterminer la quantité d'huile minimale pour recouvrir une poêle à chaud ;
- La figure 4 illustre en coupe partielle une poêle dans laquelle de l'huile recouvre le fond ;
- La figure 5 illustre un graphique de la masse d'huile nécessaire en fonction de la concavité de l'ustensile culinaire, pour un recouvrement de la surface du fond ;
- La figure 6 illustre un graphique de la masse d'huile absorbée par l'aliment cuisiné en fonction de la masse d'huile utilisée ;
- La figure 7 illustre un graphique du taux de matière grasse absorbée par rapport à la matière sèche de l'aliment en fonction de la concavité de l'ustensile culinaire.

Dans ce mode préférentiel de mise en oeuvre du procédé selon l'invention, l'ustensile culinaire utilisé est une poêle. On peut cependant mettre en oeuvre le procédé selon l'invention avec d'autres ustensiles culinaires pour la cuisson d'aliments nécessitant l'utilisation de matière grasse pour réussir convenablement ladite cuisson, lesdits ustensiles culinaires étant sujets à une déformation concave en cours de cuisson. Par exemple une casserole ou une sauteuse.

Selon l'invention, le procédé de détermination d'un indice de performance d'un ustensile culinaire pour une température de cuisson prédéterminée dans le but d'évaluer un gain nutritionnel comprend les étapes suivantes :

a) porter la surface de cuisson à ladite température de cuisson prédéterminée en utilisant des moyens de chauffe, l'échauffement de la surface entrainant une déformation du fond,

b) détermination d'une quantité de matière grasse minimale nécessaire pour recouvrir l'intégralité de la surface de cuisson du fond ainsi déformé à ladite température de cuisson prédéterminée en versant la quantité de matière grasse minimale nécessaire pour recouvrir l'intégralité de la surface de cuisson ou en calculant ladite quantité de matière grasse minimale nécessaire pour recouvrir l'intégralité de la surface de cuisson en recréant l'ustensile culinaire ainsi déformé à l'aide d'un ordinateur muni d'un logiciel de conception assistée par ordinateur.

Une poêle qui est soumise à une température de chauffe ou de cuisson, subit de fortes variations de températures qui entrainent des phénomènes de dilatation et de déformation. Ces températures n'étant pas homogènes, celles-ci entrainent des dilatations non homogènes et forcent le matériau de la poêle à se déformer. Il en est de même pour les autres ustensiles culinaires.

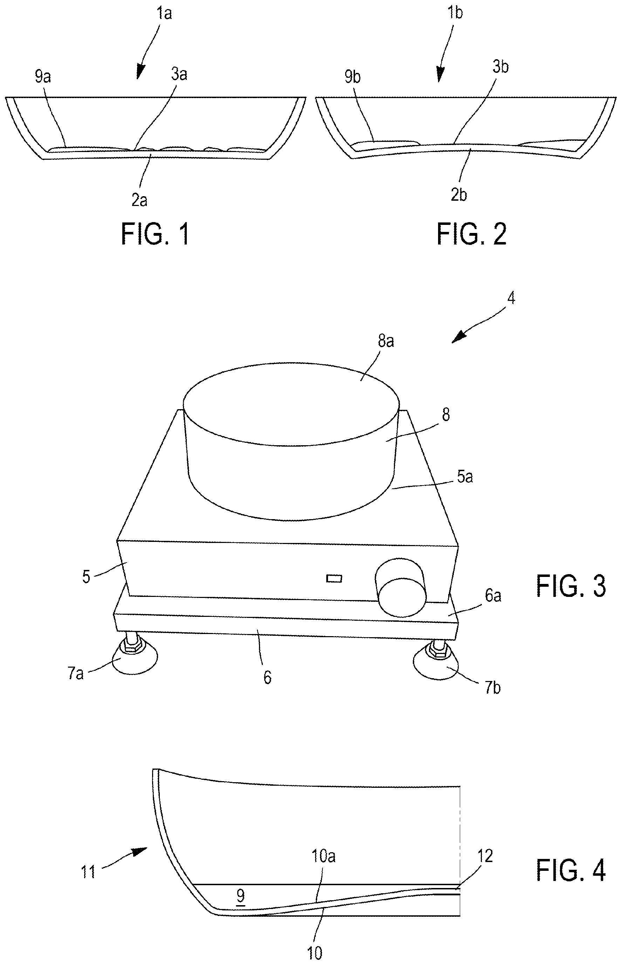

De ce fait, la concavité de la poêle peut s'accentuer voire même devenir convexe lorsqu'elle est soumise à une chauffe, cette déformation dépendant de la concavité de la poêle en sortie de fabrication et des caractéristiques techniques de fabrication de la poêle, en particulier celles concernant le fond de la poêle (matériau, grille anti-déformation, frappe...). On constate ainsi, à titre d'exemple, qu'une première poêle 1a illustrée en figure 1 présente un fond 2a avec une concavité très faible, c'est-à-dire que le fond 2a reste quasiment plat lors d'une chauffe. Tandis qu'une seconde poêle 1b illustrée en figure 2 présente un fond 2b avec une concavité fort prononcée lors d'une chauffe.

L'étape b) du procédé selon l'invention consiste en la détermination de la quantité de matière grasse minimale pour recouvrir intégralement le fond 2a, 2b de la poêle 1a, 1b. Dans le mode de réalisation décrit, la matière grasse utilisée est de l'huile. Comme on le constate sur les figures 1 et 2, la répartition de l'huile est complètement différente d'une poêle à une autre en fonction de la concavité du fond. Concernant la poêle 1a de la figure 1, qui présente un fond 2a avec une très faible concavité, l'huile 9a se répartie convenablement sur toute la surface de cuisson 3a. Au contraire, pour la poêle 1b de la figure 2, qui présente un fond 2b avec une forte concavité, l'huile 9b se positionne en périphérie de la surface de cuisson 3b. On comprend donc que pour disposer d'un mince film d'huile sur toute la surface de cuisson 3a, 3b, la quantité d'huile sera différente entre la première poêle 1a et la seconde poêle 1 b.

Pour déterminer la quantité d'huile minimale nécessaire pour recouvrir la surface de cuisson de la poêle à chaud, on utilise des moyens de chauffe 4, illustrés en figure 3, comprenant une source de chaleur 5 positionnée sur un support 6 comprenant des pieds 7a, 7b, configurés pour permettre un réglage à plat de la face supérieure 6a dudit support 6. La source de chaleur est, par exemple, une plaque radiante qui comprend un foyer 5a d'un diamètre de 21 cm. Sur ce foyer 5a est positionné un bloc en aluminium 8 répondant à la norme NF EN 12983-1. Ce bloc est mis à niveau, à plat, en procédant à un réglage du support 6. Un thermocouple (non illustré), relié à un bloc de régulation, est inséré dans la partie haute du bloc d'aluminium 8.

En outre, on utilise des poêles 1a, 1 b qui présentent un diamètre de 26 cm que l'on positionne sur la face supérieure 8a de ce bloc d'aluminium 8.

La température de cuisson prédéterminée est celle de la pomme de terre, de l'ordre de 180 à 200°C. Pour les poêles retenues la température de consigne est fixée à 235°C. Une fois que le bloc d'aluminium 8 a atteint la température de consigne de 235°C, la poêle est centrée dessus. Dès que la température à mi rayon de la surface de cuisson a atteint 180°C, on procède par étapes successives :

i. on verse une quantité d'huile correspondant à un volume de liquide de 1 mm d'épaisseur et de surface égale à un disque plan équivalent à la surface de cuisson (10a),

ii. on laisse l'huile se stabiliser à la température et se répartir sur la surface de cuisson (3a, 3b, 10a) du fond (2a, 2b, 10),

iii. on mesure une surface de cuisson résiduelle non recouverte par l'huile et on verse une quantité d'huile complémentaire correspondant à un volume de liquide de 1 mm d'épais et de surface égale à un disque plan équivalent à la surface de cuisson résiduelle

iv. on répète les étapes ii) et iii) jusqu'à recouvrir intégralement la surface de cuisson (10a) du fond (10) de l'ustensile culinaire (11).

Ainsi la somme des quantités d'huile versées dans la poêle détermine la quantité d'huile minimale pour recouvrir intégralement le fond 2a, 2b ; l'huile 9 recouvrant entièrement la surface de cuisson 10a du fond 10 de la poêle 11, comme illustré sur la figure 4. A la fin de cette étape, la poêle avec l'huile 9 recouvrant le fond 10, est pesée. La masse d'huile nécessaire à recouvrir la surface de cuisson est alors calculée par différence entre la masse de la poêle contenant l'huile et la masse de la poêle à vide. D'où la formule suivante :

$$masse_{huile} = masse_{po\hat{e}le+huile} - masse_{po\hat{e}le\ vide}$$

Bien entendu, cette masse d'huile est représentative de la quantité de matière grasse minimale.

D'autres méthodes sont envisageables pour déterminer cette quantité de matière grasse minimale nécessaire pour recouvrir la surface de cuisson de la poêle. Ainsi, il est possible de représenter graphiquement, par conception assistée par ordinateur, dite CAO, la quantité d'huile 9 minimale pour recouvrir la surface de cuisson 10a du fond 10 de la poêle 11 à chaud. Le fond 10 est considéré recouvert lorsque le film d'huile a une épaisseur minimale comprise entre 0,3 et 1 mm sur la partie centrale bombée 12 de la surface de cuisson 10a. La quantité d'huile 9 est obtenue en analysant les propriétés massiques de l'objet créé en CAO.

La température de cuisson prédéterminée est celle de la pomme de terre, dans la mesure où la cuisson de cet aliment nécessite l'utilisation de matière grasse afin de réussir convenablement la cuisson et d'améliorer ses qualités organoleptiques. Ainsi, les pommes de terre sont coupées en rondelles ou en morceaux, voire entières, selon leur grosseur, pour la mise en oeuvre d'une recette de pommes de terre sautées, couramment consommée chez les ménages. On utilise, par exemple, 800 g de pommes de terre. Cette recette nécessite une utilisation de matière grasse appropriée pour mettre en évidence tout l'intérêt du procédé selon l'invention. En effet, pour une réussite optimale, cette recette nécessite de réaliser une cuisson des pommes de terre en friture plate, avec un mince film de matière grasse permettant de supprimer les espaces d'air entre le fond de la poêle et les pommes de terre, ces espaces d'air isolant thermiquement une partie de l'aliment par rapport à la surface de cuisson. De plus, ce choix de cuisson permet de conduire plus rapidement la chaleur de la surface de cuisson aux pommes de terre, en évitant toute disparité de cuisson de ces pommes de terre.

En outre, pour la mise en oeuvre du procédé selon l'invention et de cette recette des pommes de terre sautées, la matière grasse utilisée est de l'huile d'arachide. On peut toutefois utiliser toute autre huile alimentaire, voire toute autre matière grasse se liquéfiant en totalité ou en partie une fois la température de cuisson prédéterminée atteinte, comme par exemple du beurre ou du gras de boeuf.

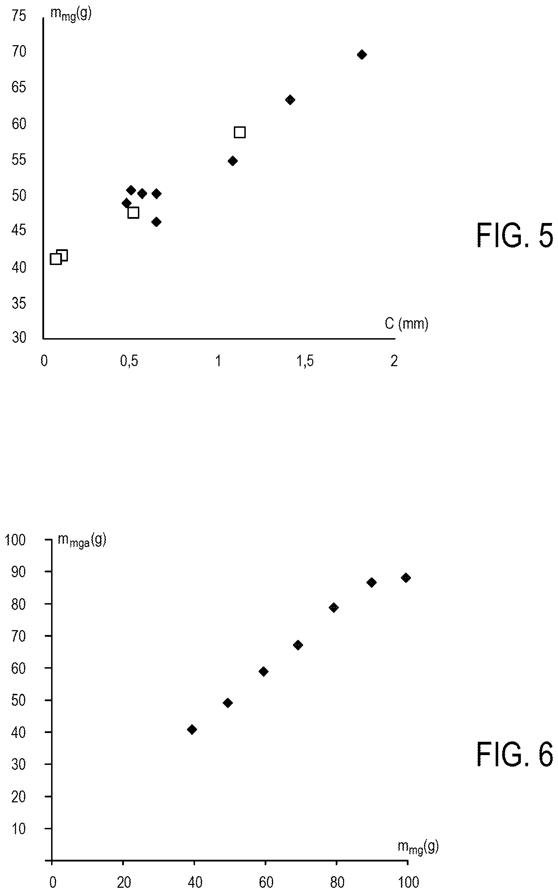

Ainsi pour un panel d'ustensiles culinaires 11 testé, notamment des poêles, dont la concavité du fond 10 varie lorsque la température en surface de cuisson atteint 180°C, on obtient le graphique de la figure 5, qui illustre la masse d'huile utilisée, $m_{mg}$, nécessaire pour recouvrir le fond 10 de la poêle 11 en fonction de la concavité C dudit fond. On constate donc que la masse d'huile utilisée $m_{mg}$ varie entre environ 40 g et 70 g pour une concavité C du fond 10 qui varie environ entre 0,1 mm, c'est-à-dire de faible concavité, et 2 mm, c'est-à-dire de forte concavité.

Ces essais ont été réalisés avec des poêles Tefal™ Grand Chef de 26 cm de diamètre et de diamètre de portée de 190 mm $\pm$ 1 mm. Soit un rapport concavité/diamètre de portée compris entre 0,0005 et 0,01.

Pour le panel de poêles retenu pour les essais, on mesure la masse de la poêle $m_{uc}$, la masse d'huile utilisée $m_{mg}$. On utilise en outre une masse de pommes de terre crues $m_{ac}$ identique et de même provenance, pour chaque cuisson. Ces mesures de masses sont réalisées au moyen d'un appareil de pesée, connu de l'homme du métier.

Le procédé comporte une étape c) qui consiste en des essais de cuisson des pommes de terre, avec le panel de poêles et la masse d'huile $m_{mg}$ nécessaire pour recouvrir la surface de cuisson 10a à chaud, prête à cuire cet aliment. Une fois la cuisson terminée, on retire la préparation de pommes de terre sautées, laquelle est pesée. On obtient donc une masse $m_{ap}$ pour les pommes de terre préparées pour le panel de poêles. De même, on mesure la masse $m_{uc+rmg}$ de la poêle et du résidu d'huile dans la poêle une fois les pommes de terre sautées préparées et retirées de cette poêle.

Ces essais permettent de constater que, plus la masse d'huile utilisée $m_{mg}$ dans la poêle est importante, plus la masse d'huile absorbée $m_{mga}$ dans la préparation de pommes de terre sautées est importante. La valeur maximale d'huile absorbée par les pommes de terre durant la cuisson, est variable. Celle-ci dépend entre autre de la variété des pommes de terre et de leur teneur en eau. D'où l'importance d'utiliser les mêmes pommes de terre pour tous les essais avec le panel de poêles.

Le procédé comporte une étape d) qui consiste à déterminer le taux de matière grasse par rapport à la matière sèche des pommes de terre $Tx_{MG/MS}$ pour le panel de poêles.

On déduit tout d'abord des mesures précédentes la masse d'huile absorbée $m_{mga}$ par les pommes de terre sautée préparées avec le panel de poêle. On utilise la formule suivante : $m_{mga} = m_{mg} - (m_{uc+rmg} - m_{uc})$

Les essais réalisés avec le panel de poêles montrent que la masse d'huile absorbée $m_{mga}$ par les pommes de terre sautées est croissante avec la masse d'huile utilisée $m_{mg}$, comme l'illustre la figure 6.

Le taux de matière grasse par rapport à la matière sèche des pommes de terre $Tx_{MG/MS}$ est alors déterminé par la formule suivante : $Tx_{MG/MS} = m_{mga}/m_{ac}/Tx_{MS}$ où $Tx_{MS}$ est le taux de matière sèche des pommes de terre.

Ce taux de matière sèche des pommes de terre est déterminé de la manière suivante. Pour un échantillon de pommes de terre identiques à celles utilisées pour les essais et, pour une masse de pommes de terre crues identique $m_{ac}$, on réalise un passage en étuve à 100°C pendant 24h puis, on mesure la masse de l'échantillon de pommes de terre après séchage $m_{acs}$. On calcule alors le taux de matière sèche : $TX_{MS} = m_{ac}/m_{acs}$

Le procédé comporte une étape qui consiste à effectuer une mesure de la déformation du fond de la poêle à chaud, notamment à la température de cuisson prédéterminée pour la cuisson d'un aliment.

Cette mesure de la déformation de la poêle à chaud est réalisée sur un panel de poêles présentant des dimensions similaires mais des caractéristiques techniques différentes qui vont donc accentuer plus ou moins ladite déformation du fond de la poêle à chaud.

La mesure de la déformation à chaud est mise en oeuvre au moyen de la méthode définie dans la norme XP D21-503 voire au moyen de tout autres méthodes connues de l'homme du métier.

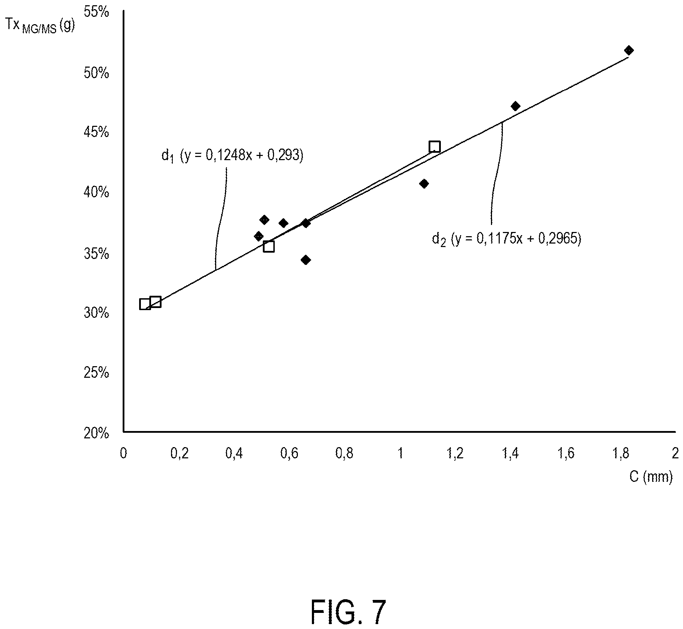

De ces essais et, à partir des mesures et calculs effectués, on aboutit au graphe de la figure 7 qui illustre le taux de matière grasse par rapport à la matière sèche des pommes de terre $Tx_{MG/MS}$ en fonction de la concavité C du fond 10 pour le panel de poêle 11 testé. Ce graphe permet d'obtenir deux droites moyennes $d_1$, $d_2$ d'équation pour $d_1$ : y=0,1248x+0,293 et, pour $d_2$ : y=0,1175x+0,2965. Soit une droite moyenne qui dispose plus ou moins d'une pente de 12%.

Ainsi, les essais montrent pour le panel de poêles en question et pour la cuisson de pommes de terre sautées qu'une diminution de 1 mm de la concavité à chaud conduit à une diminution de 12% du taux de matière grasse dans la préparation.

Dans une variante, le taux de matière grasse par rapport à la matière sèche des pommes de terre $Tx_{MG/MS}$ peut aussi être rapporté à la masse $m_{ap}$ pour les pommes de terre préparées. Cependant, la comparaison du taux de matière grasse de préparation obtenue dans deux poêles différentes devient problématique. Il faut en effet s'assurer que les deux préparations ont été cuites avec le même degré de cuisson afin d'être sûr que la perte en eau en cours de cuisson est restée identique.

Le procédé selon l'invention peut évidemment être mis en oeuvre avec un panel de poêles présentant un diamètre différent et pour une préparation alimentaire différente. Les résultats finaux étant similaires à ceux obtenus avec les essais décrits ci-dessus, à savoir que, dans tous les cas, ces essais montrent que la quantité de matière grasse absorbée par l'aliment cuisiné diminue lorsque la concavité du fond de la poêle diminue, tout en conservant des conditions de cuisson identiques pour ne pas modifier la qualité organoleptique de la préparation culinaire.

Le procédé selon l'invention trouvera son application chez les fabricants d'ustensiles pour certifier la qualité des ustensiles culinaires pour la cuisson d'aliments, tels que des poêles, des sauteuses ou autres, mis en vente dans le commerce. En effet, le procédé selon l'invention contribue à une optimisation du gain nutritionnel de par la maîtrise à chaud de la concavité du fond des ustensiles culinaires que chercheront à atteindre les fabricants en vue d'une évaluation favorable de leurs produits, au moyen dudit procédé.

## Revendications

**1.** Procédé de détermination d'un indice de performance d'un ustensile culinaire (1a, 1b, 11) pour une température de cuisson prédéterminée dans le but d'évaluer un gain nutritionnel, ledit ustensile culinaire comprenant un fond (2a, 2b, 10) présentant une surface de cuisson (3a, 3b, 10a), **caractérisé en ce qu'**il comprend les étapes suivantes :

a) porter la surface de cuisson (3a, 3b, 10a) à ladite température de cuisson prédéterminée en utilisant des moyens de chauffe (4), l'échauffement de la surface entrainant une déformation du fond (2a, 2b, 10) ;
b) détermination d'une quantité de matière grasse (9, 9a, 9b) minimale nécessaire pour recouvrir l'intégralité de la surface de cuisson (3a, 3b, 10a) du fond (2a, 2b, 10) ainsi déformé à ladite température de cuisson prédéterminée en versant la quantité de matière grasse (9, 9a, 9b) minimale nécessaire pour recouvrir l'intégralité de la surface de cuisson (3a, 3b, 10a) ou en calculant ladite quantité de matière grasse (9, 9a, 9b) minimale nécessaire pour recouvrir l'intégralité de la surface de cuisson (3a, 3b, 10a) en recréant l'ustensile culinaire (11) ainsi déformé à l'aide d'un ordinateur muni d'un logiciel de conception assistée par ordinateur, ladite quantité de matière grasse (9, 9a, 9b) minimale permettant de définir l'indice de performance de l'ustensile culinaire (1a, 1b, 11) pour une température de cuisson prédéterminée.

**2.** Procédé selon la revendication 1, caractérisé en ce pour réaliser l'étape b), on procède par étapes successives :

i. on verse une quantité de matière grasse correspondant à un volume de liquide de 1 mm d'épais et de surface égale à un disque plan équivalent à la surface de cuisson (10a),
ii. on laisse la matière grasse se stabiliser à la température et se répartir sur la surface de cuisson (3a, 3b, 10a) du fond (2a, 2b, 10),

iii. on mesure une surface de cuisson résiduelle non recouverte par la matière grasse et on verse une quantité de matière grasse correspondant à un volume de liquide de 1 mm d'épais et de surface égale à un disque plan équivalent à la surface de cuisson résiduelle,

iv. on répète les étapes ii) et iii) jusqu'à recouvrir intégralement la surface de cuisson (10a) du fond (10) de l'ustensile culinaire (11).

3. Procédé selon la revendication 1, **caractérisé en ce que** pour réaliser l'étape b), on crée l'ustensile culinaire (11) en conception assistée par ordinateur, on représente graphiquement la quantité minimale de matière grasse (9) nécessaire pour recouvrir le fond (10) de l'ustensile culinaire puis, on analyse les propriétés massiques de l'objet créé en conception assistée par ordinateur en vue de calculer ladite quantité de matière grasse nécessaire pour la cuisson.

4. Procédé selon l'une des revendications 1 à 3, **caractérisé en ce qu'**à l'étape a), la température de cuisson prédéterminée est celle de la pomme de terre, de l'ordre de 180 à 200°C.

5. Procédé selon l'une des revendications 1 à 4, **caractérisé en ce qu'**à l'étape b), la matière grasse (9, 9a, 9b) est de l'huile alimentaire.

6. Procédé selon l'une des revendications 1 à 5, **caractérisé en ce qu'**il comprend les étapes suivantes :

c) cuisson de l'aliment dans l'ustensile culinaire et quantification de la matière grasse absorbée par l'aliment cuit ;
d) détermination du taux de matière grasse absorbée par rapport à la matière sèche de l'aliment cuit.

7. Procédé selon la revendication 6, caractérisé en ce pour réaliser l'étape c) :

- on mesure la masse $m_{uc}$ de l'ustensile culinaire (11);
- on mesure la masse $m_{mg}$ de la matière grasse utilisée (9) ;
- on mesure la masse $m_{ac}$ de l'aliment cru ;
- on mesure la masse $m_{ap}$ de l'aliment préparé ;
- on mesure la masse $m_{uc+rmg}$ de l'ustensile culinaire et du résidu de matière grasse une fois l'aliment préparé, retiré de l'ustensile culinaire et ;
- on en déduit la masse $m_{mga}$ de la matière grasse absorbée par l'aliment préparé.

8. Procédé selon la revendication 7, **caractérisé en ce que** le taux de matière grasse absorbée par rapport à la matière sèche de l'aliment cuit est déterminé par la formule $Tx_{mga/ms} = m_{mga}/m_{ac}/Tx_{ms}$ où $Tx_{ms}$ est le taux de matière sèche de l'aliment.

9. Procédé selon l'une des revendications 6 à 8, **caractérisé en ce qu'**il comporte une étape de mesure de la déformation du fond de l'ustensile culinaire à la température de cuisson prédéterminée après l'étape a) et **en ce qu'**à l'étape d) on détermine le taux de matière grasse absorbée par rapport à la matière sèche de l'aliment cuit en fonction de la déformation de l'ustensile culinaire.

10. Procédé selon la revendication 9, **caractérisé en ce que** la mesure de la déformation du fond (2a, 2b, 10) de l'ustensile culinaire (1a, 1b, 11) à la température de cuisson prédéterminée est réalisée conformément à la norme XP D21-503.

11. Procédé selon l'une des revendications 1 à 10, **caractérisé en ce que** l'ustensile culinaire (1a, 1 b, 11) est une poêle.

**Patentansprüche**

1. Verfahren zur Ermittlung eines Leistungsindex von Kochgeschirr (1a, 1b, 11) für eine vorgegebene Gartemperatur mit dem Ziel, einen Ernährungsvorteil abzuschätzen, wobei das Kochgeschirr einen Boden (2a, 2b, 10) mit einer Garfläche (3a, 3b, 10a) umfasst, **dadurch gekennzeichnet, dass** es folgende Schritte umfasst:

a) Erwärmung der Garfläche (3a, 3b, 10a) mithilfe von Heizmitteln (4) auf die vorgegebene Gartemperatur, wobei die Erwärmung der Fläche eine Verformung des Bodens (2a, 2b, 10) zur Folge hat;
b) Ermittlung einer Mindestfettmenge (9, 9a, 9b), die zum Bedecken der gesamten Garfläche (3a, 3b, 10a) des so bei der vorgegebenen Gartemperatur verformten Bodens (2a, 2b, 10) erforderlich ist, indem die zum Bedecken der gesamten Garfläche (3a, 3b, 10a) erforderliche Mindestfettmenge (9, 9a, 9b) hineingegeben wird oder indem die zum Bedecken der gesamten Garfläche (3a, 3b, 10a) erforderliche Mindestfettmenge (9, 9a, 9b) berechnet wird, indem das so verformte Kochgeschirr (11) mithilfe eines mit einer Computer-Aided-Design-Software ausgestatteten Computers rekonstruiert wird, wobei die Mindestfettmenge (9, 9a, 9b) die Definition des Leistungsindex des Kochgeschirrs (1a, 1b, 11) für eine vorgegebene Gartemperatur erlaubt.

2. Verfahren nach Anspruch 1, **dadurch gekennzeichnet, dass** zur Ausführung von Schritt b) nacheinander die folgenden Schritte ausgeführt werden:

   i. eine Menge an Fett zugeben, die einem Flüssigkeitsvolumen von 1 mm Dicke und einer Fläche entspricht, die gleich einer ebenen Scheibe ist, die der Garfläche (10a) entspricht,
   ii. das Fett sich bei der Temperatur stabilisieren und sich auf der Garfläche (3a, 3b, 10a) des Bodens (2a, 2b, 10) verteilen lassen,
   iii. eine Restgarfläche messen, die nicht vom Fett bedeckt ist, und eine Menge an Fett zugeben, die einem Flüssigkeitsvolumen von 1 mm Dicke und einer Fläche entspricht, die gleich einer ebenen Scheibe ist, die der Restgarfläche entspricht,
   iv. die Schritte ii) und iii) wiederholen, bis die Garfläche (10a) des Bodens (10) des Kochgeschirrs (11) vollständig bedeckt ist.

3. Verfahren nach Anspruch 1, **dadurch gekennzeichnet, dass** zur Ausführung von Schritt b) das Kochgeschirr (11) mit computergestütztem Design rekonstruiert wird, dass die zum Bedecken des Bodens (10) des Kochgeschirrs erforderliche Mindestfettmenge (9) grafisch darstellt wird und dass dann die Masseeigenschaften des mit computergestütztem Design erstellten Objekts zwecks Berechnung der für das Garen erforderlichen Fettmenge analysiert werden.

4. Verfahren nach einem der Ansprüche 1 bis 3, **dadurch gekennzeichnet, dass** die vorgegebene Gartemperatur bei Schritt a) diejenige für Kartoffeln ist, ca. 180 bis 200 °C.

5. Verfahren nach einem der Ansprüche 1 bis 4, **dadurch gekennzeichnet, dass** das Fett (9, 9a, 9b) bei Schritt b) Speiseöl ist.

6. Verfahren nach einem der Ansprüche 1 bis 5, **dadurch gekennzeichnet, dass** es die folgenden Schritte umfasst:

   c) Garen des Lebensmittels im Kochgeschirr und Quantifizierung des vom gegarten Lebensmittel aufgenommenen Fetts;
   d) Ermittlung des Gehalts an aufgenommenem Fett in Bezug auf die Trockenmasse des gegarten Lebensmittels.

7. Verfahren nach Anspruch 6, **dadurch gekennzeichnet, dass** zur Ausführung von Schritt c):

   - die Masse $m_{uc}$ des Kochgeschirrs (11) gemessen wird;
   - die Masse $m_{mg}$ des verwendeten Fetts (9) gemessen wird;
   - die Masse $m_{ac}$ des rohen Lebensmittels gemessen wird;
   - die Masse $m_{ap}$ des zubereiteten Lebensmittels gemessen wird;
   - die Masse $m_{uc+rmg}$ des Kochgeschirrs und des Rests an Fett gemessen wird, nachdem das Lebensmittel zubereitet und aus dem Kochgeschirr genommen wurde; und
   - die Masse $m_{mga}$ des vom zubereiteten Lebensmittel aufgenommenen Fetts davon abgezogen wird.

8. Verfahren nach Anspruch 7, **dadurch gekennzeichnet, dass** der Gehalt an aufgenommenem Fett in Bezug auf die Trockenmasse des gegarten Lebensmittels mit der Formel $Tx_{mga/ms} = m_{mga}/m_{ac}/Tx_{ms}$ ermittelt wird, wobei $Tx_{ms}$ der Trockenmassegehalt des Lebensmittels ist.

9. Verfahren nach einem der Ansprüche 6 bis 8, **dadurch gekennzeichnet, dass** es einen Schritt zur Messung der Verformung des Kochgeschirrbodens bei der vorgegebenen Gartemperatur nach Schritt a) umfasst und dass bei Schritt d) der Gehalt an aufgenommenem Fett in Bezug auf die Trockenmasse des gegarten Lebensmittels entsprechend der Verformung des Kochgeschirrs ermittelt wird.

10. Verfahren nach Anspruch 9, **dadurch gekennzeichnet, dass** die Messung der Verformung des Bodens (2a, 2b, 10) des Kochgeschirrs (1 a, 1 b, 11) bei der vorgegebenen Gartemperatur entsprechend der Norm XP D21-503 erfolgt.

**11.** Verfahren nach einem der Ansprüche 1 bis 10, **dadurch gekennzeichnet, dass** das Kochgeschirr (1a, 1b, 11) eine Pfanne ist.

## Claims

**1.** Method of determining a performance index of a kitchen utensil (1 a, 1b, 11) for a predetermined cooking temperature in order to evaluate a nutritional gain, said kitchen utensil comprising a bottom (2a, 2b, 10) having a cooking surface (3a, 3b, 10a), **characterised in that** it comprises the following steps:

a) raising the cooking surface (3a, 3b, 10a) to said predetermined cooking temperature using heating means (4), heating of the surface resulting in a deformation of the bottom (2a, 2b, 10);
b) determining a minimum quantity of fat (9, 9a, 9b) necessary to cover the entire cooking surface (3a, 3b, 10a) of the bottom (2a, 2b, 10) so deformed at said predetermined cooking temperature by pouring the minimum quantity of fat (9, 9a, 9b) necessary to cover the entire cooking surface (3a, 3b, 10a) or by calculating said minimum quantity of fat (9, 9a, 9b) necessary to cover the entire cooking surface (3a, 3b, 10a) by recreating the kitchen utensil (11) so deformed using a computer equipped with computer-aided design software, said minimum quantity of fat (9, 9a, 9b) being used to define the performance index of the kitchen utensil (1a, 1b, 11) for a predetermined cooking temperature.

**2.** Method according to claim 1, **characterised in that** to perform step b), the following steps are carried out successively:

i. pouring a quantity of fat corresponding to a volume of liquid 1 mm thick and of area equal to a flat disc equivalent to the cooking surface (10a),
ii. allowing the fat to stabilise at the temperature and spread over the cooking surface (3a, 3b, 10a) of the bottom (2a, 2b, 10),
iii. measuring a residual cooking surface not covered by the fat and pouring a quantity of fat corresponding to a volume of liquid 1 mm thick and of area equal to a flat disc equivalent to the residual cooking surface,
iv. repeating steps ii) and iii) until the cooking surface (10a) of the bottom (10) of the kitchen utensil (11) is entirely covered.

**3.** Method according to claim 1, **characterised in that** to perform step b), the kitchen utensil (11) is created by computer-aided design, the minimum quantity of fat (9) necessary to cover the bottom (10) of the kitchen utensil is represented graphically, then the mass properties of the object created by computer-aided design are analysed in order to calculate said quantity of fat required for cooking.

**4.** Method according to one of claims 1 to 3, **characterised in that** in step a) the predetermined cooking temperature is that of the potato, about 180 to 200°C.

**5.** Method according to one of claims 1 to 4, **characterised in that** in step b) the fat (9, 9a, 9b) is edible oil.

**6.** Method according to one of claims 1 to 5, **characterised in that** it comprises the following steps:

c) cooking the food in the kitchen utensil and quantifying the fat absorbed by the cooked food;
d) determining the amount of fat absorbed with respect to the dry matter of the cooked food.

**7.** Method according to claim 6, **characterised in that** to perform step c):

- the mass $m_{uc}$ of the kitchen utensil (11) is measured;
- the mass $m_{mg}$ of fat used (9) is measured;
- the mass $m_{ac}$ of the raw food is measured;
- the mass $m_{ap}$ of the prepared food is measured;
- the mass $m_{uc+rmg}$ of the kitchen utensil and of the fat residue is measured once the prepared food has been removed from the kitchen utensil and;

- the mass $m_{mga}$ of the fat absorbed by the prepared food is deduced.

8. Method according to claim 7, **characterised in that** the amount of fat absorbed with respect to the dry matter of the cooked food is determined by the formula $Tx_{mga/ms} = m_{mga}/m_{ac}/Tx_{ms}$ where $Tx_{ms}$ is the dry matter content of the food.

9. Method according to one of claims 6 to 8, **characterised in that** it comprises a step of measuring the deformation of the bottom of the kitchen utensil at the predetermined cooking temperature after step a) and **in that** in step d) the amount of fat absorbed with respect to the dry matter of the cooked food is determined based on the deformation of the cooking utensil.

10. Method according to claim 9, **characterised in that** the deformation of the bottom (2a, 2b, 10) of the kitchen utensil (1a, 1b, 11) at the predetermined cooking temperature is measured in compliance with standard XP D21-503.

11. Method according to one of claims 1 to 10, **characterised in that** the kitchen utensil (1 a, 1 b, 11) is a frying pan.

1a

9a 3a

2a

## FIG. 1

1b

9b 3b

2b

## FIG. 2

4

8a

8

5a

5

6a

7a

6

7b

## FIG. 3

11

10a

9

10

12

## FIG. 4

FIG. 5

FIG. 6

FIG. 7

**EP 2 975 983 B1**

## RÉFÉRENCES CITÉES DANS LA DESCRIPTION

### Documents brevets cités dans la description

- FR 2711050
- FR 2780626
- FR 2919484
- US 20100224636 A1